(19) Europäisches Patentamt
European Patent Office
Office européen des brevets

(11) Publication number: **0 114 331**
**B1**

(12) # EUROPEAN PATENT SPECIFICATION

(45) Date of publication of patent specification: **20.05.87**

(51) Int. Cl.$^4$: **C 07 H 19/24** // C07H19/04

(21) Application number: **83112753.5**

(22) Date of filing: **19.12.83**

(54) **A process for preparing oxanosine.**

(30) Priority: **22.12.82 JP 223991/82**

(43) Date of publication of application:
**01.08.84 Bulletin 84/31**

(45) Publication of the grant of the patent:
**20.05.87 Bulletin 87/21**

(84) Designated Contracting States:
**AT BE CH DE FR GB IT LI LU NL SE**

(56) References cited:
**EP-A-0 053 025**

**"THE CARBOHYDRATES", second edition, vol. IIA, 1970, pages 16-17, ed. W. Pigman, pub. Academic Press, New York, London C.A. DEKKER et al.: "Synthesis by construction of a heterocyclic base after glycosylation"**

**TETRAHEDRON LETTERS, vol. 24, no. 9, 1983, pages 931-932, Pergamon Press, Ltd., GB N. YAGISAWA et al.: "A facile total synthesis of oxanosine, a novel nucleoside antibiotic"**

**CHEMICAL ABSTRACTS, vol. 100, no. 11, 12th March 1984, abstract no. 82459b, Columbus, Ohio, US N. YAGISAWA et al.: "Structure and total synthesis of oxanosine, a nucleoside antibiotic"**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao, Prof.**
**4-23, Toyotama-kita Nerima-ku**
**Tokyo (JP)**
Inventor: **Takeuchi, Tomio**
**5-1-11, Higashi-Gotanda Shinagawa-ku**
**Tokyo (JP)**
Inventor: **Takita, Tomohisa**
**7-10-15, Negishidai**
**Asaka city Saitama Prefecture (JP)**
Inventor: **Shimada, Nobuyoshi**
**2-9-10, Shimo Kita-ku**
**Tokyo (JP)**
Inventor: **Katoh, Kuniki**
**5-24-4, Ohto**
**Yono city Saitama Prefecture (JP)**
Inventor: **Yagisawa, Naomasa**
**1-9-44, Miyamae Miyamae-ku**
**Kawasaki city Kanagawa Prefecture (JP)**

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

**EP 0 114 331 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the synthesis of oxanosine, an antibiotic having carcinostatic activity.

Oxanosine (5-amino-3-β-D-ribofuranosyl-3H-imidaso[4,5-d][1,3]oxazin-7-one) was isolated by Hamao Umezawa et al from culture broth of Streptomyces capreolus MG265—CF3 (Deposit No. 5735 at the Fermentation Research Institute, Agency of Industrial Science and Technology, Ministry of International Trade and Industry of Japan) (see the Journal of Antibiotics, Vol. 34, p. 1216—1218, 1981).

We, the inventors of this invention, made extensive studies on how to synthesize oxanosine, and found out that oxanosine expressed by the formula (II)

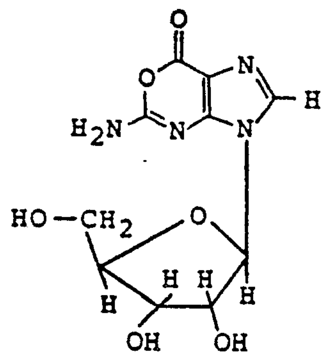

(II)

can be synthesized in the manner described below. A 5-amino-1-(o-protected-β-D-ribofuranosyl)-4-imidazolecarboxylic acid alkyl ester of the general formula (III)

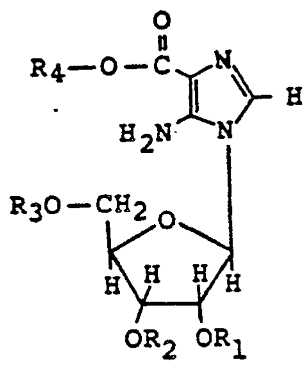

(III)

wherein $R_1$ represents a protective group; $R_2$ and $R_3$ each represent hydrogen or a protective group, or $R_1$ and $R_2$ may be bound together to form a ring, and $R_4$ represents lower alkyl is condensed with a carbonyl isothiocyanate of the general formula (IV)

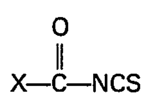

(IV)

wherein X represents a lower alkoxy group or a phenyl group, to form a 5-substituted amino-1-(o-protected-β-D-ribofuranosyl)-4-imidazolecarboxylic acid alkyl ester of the general formula (V)

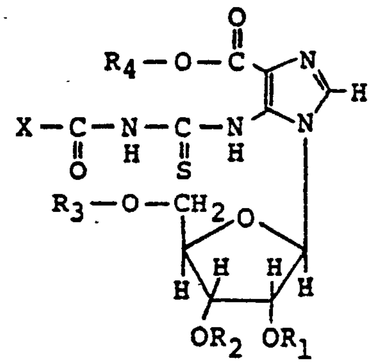

(V)

wherein $R_1$, $R_2$, $R_3$, $R_4$ and X are as defined above.

The resulting compound is subjected to the action of an alkyl iodide to form an s-alkyl derivative of the general formula (VI)

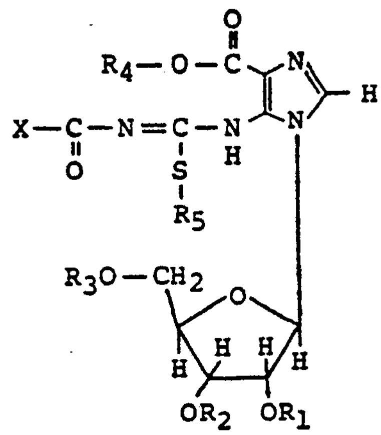

(VI)

wherein $R_1$, $R_2$, $R_3$, $R_4$ and X are as defined above, and $R_5$ represents a lower alkyl group.

This compound is heated in the presence of an alkali metal hydroxide such as sodium hydroxide or potassium hydroxide, or an alkali metal carbonate such as sodium carbonate or potassium carbonate) to form a 5-cyanamide-1-(o-protected-β-D-ribofuranosyl)-4-imidazolecarboxylic acid metal salt of the formula (VII)

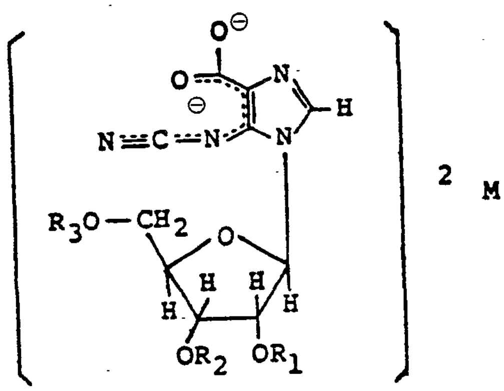

(VII)

wherein M represents an alkali metal atom.

Neutralization of the compound of the formula (VII) gives a 2′-o-protected oxanosine of the general formula (I):

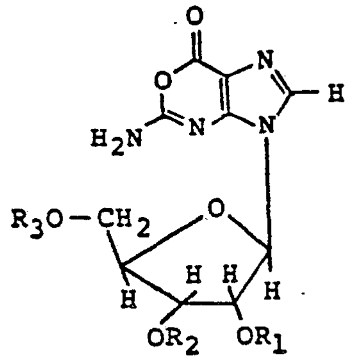

(I)

wherein $R_1$, $R_2$ and $R_3$ are as defined above.

The oxanosine of the formula (II) is obtained by hydrolyzing the 2′-O-protected oxanosine of the formula (I) above, thereby eliminating the protective group(s).

The compound of the general formula (III), in which $R_4$ represents an ethyl group, $R_3$ is a hydrogen atom, and $R_1$ and $R_2$ are bonded together to form an isopropylidene group, i.e., 5-amino-1-(2,3-o-isopropylidene-β-D-ribofuranosyl)-4-imidazolecarboxylic acid ethyl ester, is a known compound synthesized by N. J. Cusack et al (Journal of Chemical Society, Part I, 1973 Issue, p. 1720, 1973). Other compounds expressed by the general formula (III) can be synthesized in accordance with the method described in this literature. Examples of the lower alkyl group in the present invention are methyl, ethyl, propyl and butyl.

Examples of the carbonyl isothiocyanate of the general formula (IV) include methoxycarbonyl isothiocyanate, ethoxycarbonyl isothiocyanate, propoxycarbonyl isothiocyanate and benzoyl isothiocyanate. In the case of benzoyl isothiocyanate, a substituent which will exert no adverse influence on the reaction may be present on the phenyl group.

The condensation between the compound of the formula (III) and the compound of the formula (IV) can be carried out, at a temperature of 0 to 40°C, preferably, room temperature, usually, in an organic solvent, e.g., a polar organic solvent such as dimethylformamide.

$R_1$ in the general formula (I) of this invention may be any protective group which can be used to protect a hydroxyl group. Examples include a tetrahydropyranyl group, a 4-methoxytetrahydropyran-4-yl group, a tetrahydrothiofuranyl group and a tetrahydrothiopyranyl group. Preferred examples of $R_1$ are those bound to $R_2$ to form a ring, which serves as a ketal-type protective group. Examples of the protective group resulting from ring formation are substituted or unsubstituted alkylidene groups, such as an isopropylidene group, a p-anisylidene group, a benzylidene group, a cyclohexylidene group, an ethylidene group and a methoxyethylidene group.

The above-mentioned elimination of the protective group(s) by hydrolysis can be performed by a customary method. If the protective group is a tetrahydropyranyl group or one of a ketal type, such as an isopropylidene group, for example, it is preferred to eliminate it with heating (say, at a temperature of 40°C or higher, preferably, under reflux) in the presence of an acid such as hydrochloric acid or acetic acid.

The present invention will be described in more detail by reference to the following examples.

## Example 1

Conversion of 2′,3′-O-isopropylideneoxanosine into oxanosine

10.0 mg of 2′,3′-O-isopropylideneoxanosine was dissolved in 1 ml of a 10% aqueous solution of acetic acid, and the resulting solution was refluxed for 1.5 hours. The solution was diluted with 15 ml of water, and the dilution was adsorbed onto 1 ml of activated carbon. The adsorbate was eluted with a 70% aqueous solution of acetone. The eluate was concentrated under reduced pressure to remove acetone. The residual aqueous solution gave 7.2 mg of oxanosine as colorless platey crystals. The yield was 84.4%, and the decomposition point, 197—199°C.

The 2′,3′-O-isopropylidene-oxanosine can be obtained by the following reaction steps:

(a) Synthesis of 5-(N′-ethoxycarbonylthiocarbamoyl)amino-1-(2,3-O-isopropylidene-β-D-ribofuranosyl)-4-imidazolecarboxylic acid ethyl ester.

The 76.0 mg of 5-amino-1-(2,3-O-isopropylidene-β-D-ribofuranosyl)-4-imidazolecarboxylic acid ethyl ester was dissolved in 1 ml of dimethylformamide. To the solution was added 0.02 ml of ethoxycarbonyl isothiocyanate, and the mixture was stirred at room temperature for 16 hours. Then, the mixture was concentrated to dryness, and the residue was dissolved in a small amount of ethyl alcohol. The solution was purified by preparative thin-layer chromatography on silica gel using benzene-ethyl alcohol (10:1) as a solvent system. The 84.8 mg of 5-(N′-ethoxycarbonylthiocarbamoyl)amino-1-(2,3-O-isopropylidene-β-D-ribofuranosyl)-4-imidazolecarboxylic acid ethyl ester was obtained as a lyophilized powder.

Yield: 81.9%

m.p.: 95.5—99.0°C (in a sealed tube)

Specific rotation: $[\alpha]_D^{18} = -47.7°$ (c = 1, $CHCl_3$)
Molecular formula: $C_{18}H_{26}N_4O_8S$
Molecular weight: 458.50
UV spectrum: $\lambda_{max}^{MeOH}$ nm (log ε):
289 (4.24), 248 (4.62), 212 (4.67)
IR spectrum: $\nu_{KBr}^{C=O}$: 1710 $cm^{-1}$
NMR spectrum for proton (chloroform-d):
8.00 ppm (s, 1H), 5.9 (d, 1H), 4.96 (d, 2H), 4.30 (m, 5H), 3.80 (m, 2H), 1.50 (s, H), 1.30 (m, 9H),

(b) Synthesis of 5-(N'-ethoxycarbonyl-S-methylisothiocarbamoyl)amino-1-(2,3-O-isopropylidene-β-D-ribofuranosyl)-4-imidazolecarboxylic acid ethyl ester

The 8.48 mg of 5-(N'-ethoxycarbonylthiocarbamoyl)amino-1-(2,3-O-isopropylidene-β-D-ribofuranosyl)-4-imidazolecarboxylic acid ethyl ester was dissolved in 1.85 ml of an aqueous solution of 0.1N sodium hydroxide. To the solution was added 0.013 ml of methyl iodide, followed by stirring the mixture for 16 hours at room temperature. Then, the mixture was diluted with 30 ml of water, and the dilution was adsorbed onto 5 ml of activated carbon. The adsorbate was eluted with a 70% aqueous solution of acetone. The eluate gave 46.9 mg of 5-(N'-ethoxycarbonyl-S-methylisothiocarbamoyl)amino-1-(2,3-O-isopropylidene-β-D-ribofuranosyl)-4-imidazolecarboxylic acid ethyl ester as a glassy substance.
Yield: 55.0%
m.p.: 64.5—67.5°C
Specific rotation: $[\alpha]_D^{18} = -26.0°$ c = 1, $CHCl_3$)
Molecular formula: $C_{19}H_{28}N_4O_8S$
Molecular weight: 472.52
UV spectrum: $\lambda_{max}^{MeOH}$ nm (log ε):
267 (4.27), 253 (4.26), 212 (4.41)
IR spectrum: $\nu_{KBr}^{C=O}$: 1750 $cm^{-1}$
NMR spectrum for proton (chloroform-d):
7.85 (s, 1H), 5.70 (d, 1H), 4.95 (d, 2H), 4.20 (m, 5H), 3.85 (m, 2H), 2.46 (s, 3H), 1.55 (s, 3H), 1.25 (m, 9H).

(c) Synthesis of 2',3'-O-isopropylideneoxanosine

The 42.3 mg of 5-(N'-ethoxycarbonyl-S-methylisothiocarbamoyl)amino-1-(2,3-O-isopropylidene-β-D-ribofuranosyl)-4-imidazole carboxylic acid ethyl ester was dissolved in 2.5 ml of an aqueous solution of 0.2N sodium hydroxide. The solution was refluxed for 30 minutes, and immediately thereafter, it was lyophilized to obtain a powder. The IR spectrum of the powder showed an absorption band for nitrile at 2140 $cm^{-1}$, which confirmed the formation of the disodium salt of 5-cyanamido-1-(2,3-O-isopropylidene-β-D-ribofuranosyl)-4-imidazolecarboxylic acid. This powder was dissolved in 0.1N hydrochloric acid for neutralization, and adsorbed onto 20 ml of activated carbon. The adsorbate was eluted with a 70% aqueous solution of acetone, and the eluate was concentrated to dryness. The residue was dissolved in a small amount of ethyl alcohol, and the solution was purified by preparative thin-layer chromatography on silica gel using ethyl acetate-methyl alcohol (9:1) as a solvent system. Crystallization from hot water gave 5.5 mg of 2',3'-O-isopropylideneoxanosine as colorless needles.
Yield: 18.5%
m.p.: 166.0—167.5°C
Mixed m.p. of this product and the authentic substance obtained from oxanosine: 166.0—167.5°C
Specific rotation: $[\alpha]_D^{18} = -34°$ (c = 0.5, MeOH)
Molecular formula: $C_{13}H_{16}N_4O_6$
Molecular weight: 324.30
UV spectrum: $\lambda_{max}^{MeOH}$ nm (log ε):
285 (4.03), 246 (4.19), 207 (4.28).
IR spectrum: $\nu_{KBr}^{C=O}$: 1770 $cm^{-1}$
NMR spectrum for proton (methanol-$d_4$):
7.96 ppm (s, 1H), 5.97 (d, 1H), 5.18 (d, d, 1H), 5.00 (d, d, 1H), 4.30 (m, 1H), 3.70 (d, 2H), 1.55 (s, 3H), 1.37 (s, 3H).

A further method for the preparation of 2',3'-O-isopropylideneoxanosine comprises the following steps:

(d) Synthesis of 5-(N'-benzoylthiocarbamoyl)amino-1-(2,3-O-isopropylidene-β-D-ribofuranosyl)-4-imidazolecarboxylic acid ethyl ester

The 30.7 mg of 5-amino-1-(2,3-O-isopropylidene-β-D-ribofuranosyl)-4-imidazolecarboxylic acid ethyl ester was dissolved in 1 ml of dimethyl sulfoxide. A 0.3 ml of benzoyl isothiocyanate was added to the solution, and the mixture was reacted for 16 hours at room temperature. The reaction mixture was concentrated under reduced pressure, and the oily residue was dissolved in a small amount of ethyl alcohol. The solution was purified by preparative thin-layer chromatography using benzene-ethyl alcohol (10:1) as a solvent system. The purified material gave 31.2 mg of 5-(N'-benzoylthiocarbamoyl)amino-1-(2,3-O-isopropylidene-D-ribofuranosyl)-4-imidazolecarboxylic acid ethyl ester as an amorphous powder.
Yield: 67.8%.

(e) Synthesis of 5-(N'-benzoyl-S-methylisothiocarbamoyl)amino-1-(2,3-O-isopropylidene-β-D-ribofuranosyl)-4-imidazolecarboxylic acid ethyl ester

The 30.0 mg of 5-(N'-benzoylthiocarbamoyl)amino-1-(2,3-O-iso-propylidene-β-D-ribofuranosyl)-4-imidazolecarboxylic acid ethyl ester was dissolved in 0.6 ml of an aqueous solution of 0.1N sodium hydroxide. A 0.01 ml of methyl iodide was added to the solution, and the mixture was stirred for 3 hours at room temperature. Crystals formed were collected by filtration, washed with water, and dried to obtain 22.3 mg of 5-(N'-benzoyl-S-methylisothiocarbamoyl)amino-1-(2,3-O-isopropylidene-β-D-ribofuranosyl)-4-imidazolecarboxylic acid ethyl ester in 72.3% yield.

(f) The resulting product was cyclized in accordance with the procedure of step (c) to obtain 2',3'-O-isopropylideneoxanosine.

**Claim**

A process for synthesizing oxanosine of the formula (II)

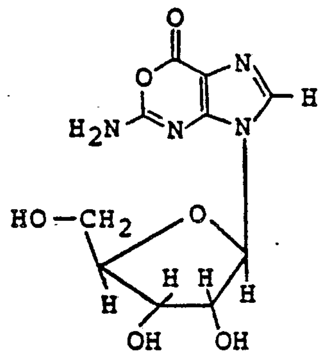

(II)

which comprises heating a compound of the formula (VI)

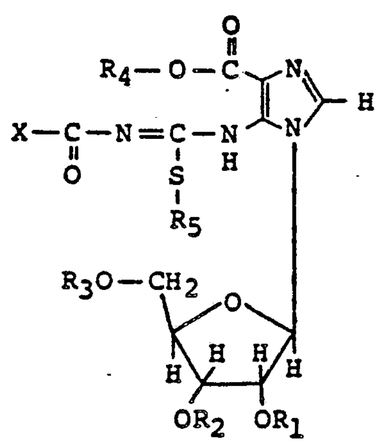

(VI)

wherein X represents a lower alkoxy group or a phenyl group, $R_1$ represents a protective group; $R_2$ and $R_3$ each represent hydrogen or a protective group and $R_1$ and $R_2$ may be bound together to form a ring and $R_4$ and $R_5$ represents lower alkyl, in the presence of an alkali metal hydroxide or carbonate to form a salt of the formula (VII)

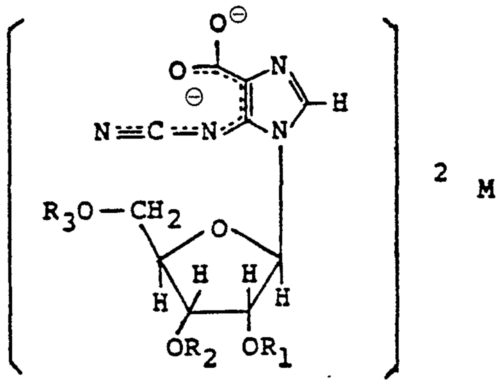

(VII)

wherein M represents an alkali metal atom, neutralizing the salt obtained to form a compound of the formula (I)

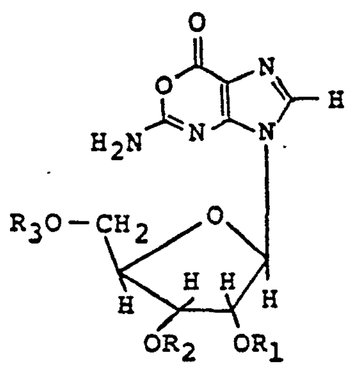

(I)

wherein $R_1$, $R_2$ and $R_3$ are defined as above and finally eliminating the protective group(s) from the compound obtained by hydrolyzation.

**Patentanspruch**

Verfahren zur Synthese von Oxanosin der Formel (II)

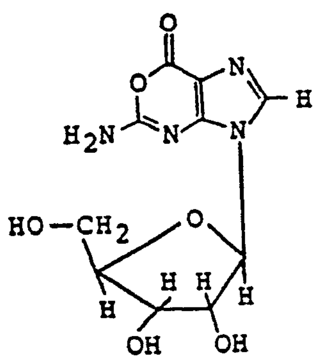

(II)

dadurch gekennzeichnet, daß man eine Verbindung der Formel (VI)

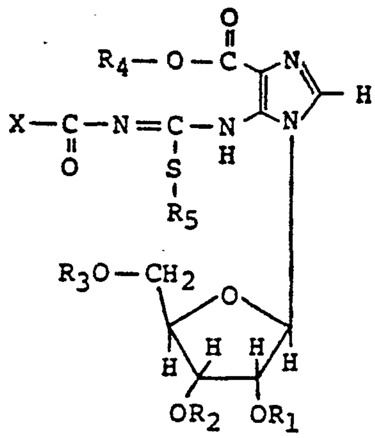

(VI)

worin X eine niedere Alkoxygruppe oder eine Phenylgruppe bedeutet, $R_1$ für eine Schutzgruppe steht, $R_2$ und $R_3$ jeweils Wasserstoff oder eine Schutzgruppe bedeuten, $R_1$ und $R_2$ ringförmig verbunden sein können und $R_4$ und $R_5$ niederes Alkyl bedeuten, in Gegenwart eines Alkalihydroxids oder -carbonats erhitzt, wobei man ein Salz der Formel (VII)

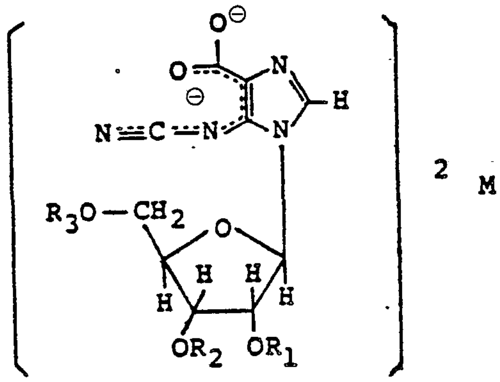

(VII)

erhält, worin M ein Alkalimetall bedeutet, das erhaltene Salz zu einer Verbindung der Formel (I)

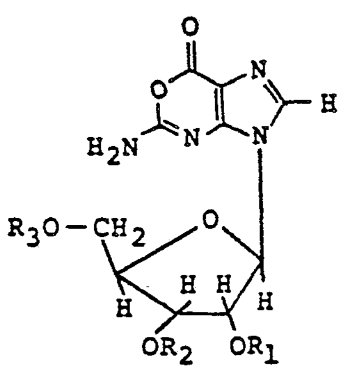

(I)

worin $R_1$, $R_2$ und $R_3$ die obige Bedeutung besitzen, neutralisiert und schließlich die Schutzgruppe(n) aus der erhaltenen Verbindung hydrolytisch entfernt.

**Revendication**

Procédé pour la synthèse de l'oxanosine de formule (II)

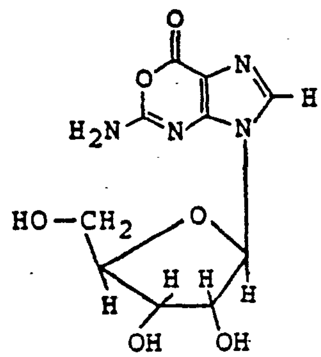

(II)

qui consiste à chauffer un composé de formule (VI)

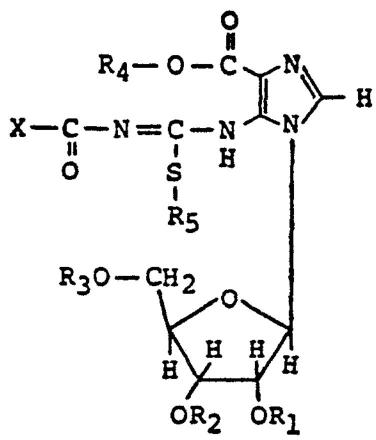

(VI)

dans laquelle X représente un groupe alcoxy inférieur ou un groupe phényle; $R_1$ représente un groupe protecteur; $R_2$ et $R_3$ représentent chacun l'hydrogène ou un groupe protecteur et $R_1$ et $R_2$ peuvent être reliés ensemble de manière à former un noyau, et $R_4$ et $R_5$ représentent un groupe alkyle inférieur, en présence d'un hydroxyde ou d'un carbonate de métal alcalin, de manière à former un sel de formule (VII)

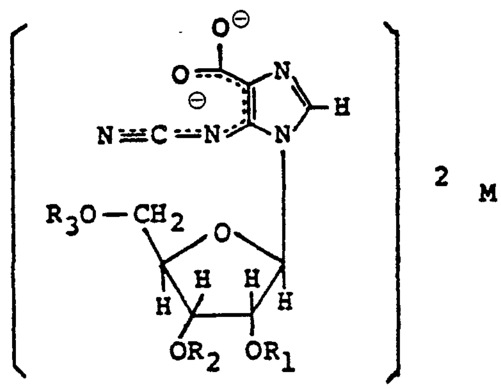

(VII)

dans laquelle M représente un atome de métal alcalin; puis à neutraliser le sel obtenu pour former un composé de formule (I)

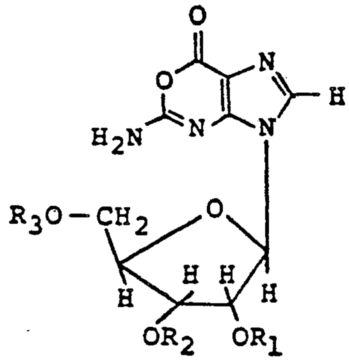

(I)

dans laquelle $R_1$, $R_2$ et $R_3$ sont tels que définis ci-dessus, et enfin à éliminer le ou les groupes protecteurs à partir du composé obtenu par hydrolyse.